# EUROPEAN PATENT APPLICATION

(11) **EP 3 508 851 A2**
(43) Date of publication of application: **10.07.2019**
(21) Application number: 17846690.0
(22) Date of filing: 31.08.2017
(51) Int. Cl.: G01N 33/543, C09K 11/06, G01N 21/64

(54) **FLUORESCENT PARTICLES FOR DIAGNOSTIC AGENT AND IMMUNOASSAY REAGENT USING SAME**

(30) Priority: 31.08.2016 JP 2016168791
(71) Applicant: Sekisui Chemical Co., Ltd., Osaka-shi, Osaka 530-8565 (JP); Sekisui Medical Co., Ltd., Tokyo 103-0027 (JP)
(72) Inventor: IWAMOTO, Tadashi, Mishima-gun Osaka 618-0021 (JP); SUGIMOTO, Satoru, Mishima-gun Osaka 618-0021 (JP); WAKIYA, Takeshi, Mishima-gun Osaka 618-0021 (JP); KITAHARA, Shinichiro, Tokyo 103-0027 (JP); YAJI, Maasa, Tokyo 103-0027 (JP)
(74) Representative: Hoffmann Eitle
(86) International application number: PCT/JP2017/031499
(87) International publication number: WO 2018/043687

(57) **Abstract**

The present invention provides fluorescent particles for a diagnostic agent which contain a synthetic polymer and at least 10 mass%, on a total particle mass basis, of an aggregation-induced emission material, and have on the surface thereof a binding partner which binds with an analyte. These fluorescent particles for a diagnostic agent are non-toxic, have improved visibility and reproducibility, and support a binding partner for an analyte.

## Description

### TECHNICAL FIELD

The present invention relates to fluorescent particles for a diagnostic agent and an immunoassay reagent using the same.

### BACKGROUND ART

Currently, in clinical test reagents (hereinafter, referred to as diagnostic reagents), many assay reagents by immunochromatography using carrier microparticles carrying binding partners (e.g., antibodies, etc.) for analytes have been practically used. In order to improve detection sensitivity of analytes, it has been proposed to use fluorescent particles that emit light by light irradiation as carrier microparticles (for example, see Patent Literature 1) .

Patent Literature 1 discloses silica particles containing an organic fluorescent material as fluorescent particles used for immunochromatography. Since the particle surface of the silica particles is hydrophilic, nonspecific reactions caused by hydrophobic interaction can be suppressed. On the other hand, when a binding partner for an analyte is supported, it is difficult to use a physical adsorption method, and it is necessary to use a chemical binding method.

Patent Literature 2 discloses a core/shell type microparticle phosphor substantially composed of inorganic fluorescent microparticles. These inorganic fluorescent microparticles have microparticle properties suitable for biotechnology fields, such as antigen-antibody reaction, and an excitation wavelength suitable for fluorescence observation, but there is a problem of toxicity because they are inorganic microparticles. There is also a problem in dispersibility.

Further, Patent Literature 3 discloses fluorescence-emitting microparticles substantially composed of organic fluorescent microparticles and a preparation method thereof, although they are not microparticles intended to be applied to biotechnology fields. According to these organic fluorescent microparticles, the above toxicity problem is solved, but a problem remains in light emission intensity or the like, when the microparticles are used as a material for a diagnostic agent.

### CITATION LIST

### PATENT LITERATURE

Patent Literature 1: International Publication No. WO 2015-022914
Patent Literature 2: International Publication No. WO 2007-102458
Patent Literature 3: Japanese Patent Laid-Open No.2003-313545

### SUMMARY OF INVENTION

### TECHNICAL PROBLEM

Accordingly, there is a demand for microparticles capable of easily supporting a binding partner for an analyte, having no toxicity, and having analyte detection sensitivity (particularly, visibility) and detection reproducibility, when used as a material for a diagnostic agent.

An object of the present invention is to provide fluorescent particles for a diagnostic agent supporting a binding partner for an analyte, the fluorescent particles capable of easily supporting the binding partner for the analyte, having no toxicity, and having improved detection sensitivity (particularly, visibility) and detection reproducibility, when used as a material for a diagnostic agent, and an assay reagent using the fluorescent particles.

### SOLUTION TO PROBLEM

The present invention includes the following descriptions:
(1) Fluorescent particles for a diagnostic agent, the fluorescent particles including a synthetic polymer and at least 10 mass%, on a total particle mass basis, of an aggregation-induced emission material, and having on the surface thereof a binding partner which binds with an analyte.
(2) The fluorescent particles for a diagnostic agent of (1), wherein the particles have a CV value of 20% or less and an average particle diameter of 0.05 µm to 3.0 µm.
(3) The fluorescent particles for a diagnostic agent of (1) or (2), wherein the synthetic polymer is a copolymer including at least one selected from the group consisting of a styrene monomer and (meth)acrylic acid.
(4) The fluorescent particles for a diagnostic agent of any one of (1) to (3), wherein a number average molecular weight of the aggregation-induced emission material is 10, 000 or less.
(5) The fluorescent particles for a diagnostic agent of any one of (1) to (4), wherein the aggregation-induced emission material is an ethylene derivative or a benzene derivative substituted with four or more phenyl groups or phenyl group derivatives.
(6) The fluorescent particles for a diagnostic agent of any one of (1) to (4), wherein the aggregation-induced emission material is selected from the group consisting of tetraphenylethylene, 1-(4-bromophenyl)-1,2,2-triphenyl ethylene, and tetrakis(4-hydroxyphenyl)ethylene.
(7) The fluorescent particles for a diagnostic agent of any one of (1) to (4), wherein the aggregation-induced emission material is hexaphenylsilole or hexaphenylbenzene.
(8) An immunoassay reagent using the fluorescent particles for a diagnostic agent of any one of (1) to (7).
(9) A method of preparing aggregation-induced emission material-containing particles, the method including:
   preparing a seed particle dispersion in which seed particles containing a synthetic polymer are dispersed,
   preparing an emulsion containing an aggregation-induced emission material and an organic solvent,
   obtaining a swollen particle droplet dispersion by adding the emulsion to the seed particle dispersion and absorbing the aggregation-induced emission material and the organic solvent into the seed particles, and
   obtaining the aggregation-induced emission material-containing particles by in-liquid drying the organic solvent in the swollen particle droplets.
(10) The method of preparing aggregation-induced emission material-containing particles of (9), wherein the emulsion is added to the seed particle dispersion such that the mass of the aggregation-induced emission material is 0.1 time to 64 times the mass of the seed particles.
(11) A method of measuring an analyte, the method including:
   preparing a mixed solution by mixing a sample solution possibly containing an analyte and a solution containing aggregation-induced emission fluorescent material-containing particles having a binding partner for the analyte;
   developing the mixed solution on an insoluble carrier having at least one detection portion on which binding partners for the analyte are immobilized;
   measuring the fluorescence intensity generated from the aggregation-induced emission fluorescent material-containing particles in the detection portion; and
   comparing a fluorescence intensity calibration curve for an analyte concentration with the fluorescence intensity, and associating the fluorescence intensity with the analyte concentration in the mixed solution.
(12) An immunochromatographic test strip, including (a) a supplying portion of a sample solution that possibly contains an analyte, (b) a conjugate pad that contains aggregation-induced emission fluorescent material-containing particles having on the surface thereof binding partners for the analyte, and (c) an insoluble membrane carrier having at least one detection portion on which binding partners for the analyte are immobilized, wherein the aggregation-induced emission fluorescent material-containing particles contain (i) a synthetic polymer and at least 10 mass%, on a total particle mass basis, of (ii) an aggregation-induced emission material.
(13) The test strip of (12), wherein the aggregation-induced emission fluorescent material-containing particles have a CV value of 20% or less and an average particle diameter of 0.05 µm to 3.0 µm.
(14) The test strip of (12) or (13), wherein the synthetic polymer contained in the aggregation-induced emission fluorescent material particles is a copolymer including at least one selected from the group consisting of a styrene monomer and (meth)acrylic acid.
(15) The test strip of any one of (12) to (14), wherein a number average molecular weight of the aggregation-induced emission fluorescent material contained in the aggregation-induced emission material particles is 10,000 or less.
(16) The test strip of any one of (12) to (15), wherein the aggregation-induced emission material contained in the aggregation-induced emission fluorescent material particles is an ethylene derivative or a benzene derivative substituted with four or more phenyl groups or phenyl group derivatives.
(17) The test strip of any one of (12) to (16), wherein the aggregation-induced emission fluorescent material contained in the aggregation-induced emission material particles is selected from the group consisting of tetraphenylethylene, 1-(4-bromophenyl)-1,2,2-triphenyl ethylene, and tetrakis(4-hydroxyphenyl)ethylene.
(18) The test strip of any one of (12) to (17), wherein the aggregation-induced emission material contained in the aggregation-induced emission fluorescent material particles is hexaphenylsilole or hexaphenylbenzene.

### EFFECTS OF INVENTION

According to the present invention, provided is fluorescent particles for a diagnostic agent supporting a binding partner for an analyte, the fluorescent particles having no toxicity and having improved analyte detection sensitivity (particularly, visibility) and detection reproducibility.

### BRIEF DESCRIPTION OF DRAWINGS

FIG. 1 is an SEM image of the surface of aggregation-induced emission fluorescent material-containing particles of the present invention;
FIG. 2 is a photograph showing aggregating luminescence when the aggregation-induced emission material-containing particles of the present invention are irradiated with ultraviolet rays (UV) of a wavelength of 365 nm;
FIG. 3 is a partially enlarged view of FIG. 2;
FIG. 4 is a photograph showing aggregating luminescence when aggregation-induced emission material-containing particles of Comparative Example 1 are irradiated with ultraviolet rays (UV) of a wavelength of 365 nm;
FIG. 5 is a partially enlarged view of FIG. 4; and
FIG. 6 is a conceptual diagram of an immunochromatographic test strip.

### DESCRIPTION OF EMBODIMENTS

Hereinafter, the present invention will be described with reference to embodiments, but the present invention is not limited to the following embodiments.

### [Fluorescent Particles for Diagnostic Agent]

As a result of extensive studies to solve the above problems, the present inventors have found that fluorescent particles having a high light emission intensity may be obtained by including a high content of a fluorescent material (hereinafter, referred to as "aggregation-induced emission material") in polymer microparticles, wherein the fluorescent material exhibits a high light emission property by aggregating in spite of hardly emitting light in a dispersed state in a solution. That is, the present invention relates to fluorescent particles for a diagnostic agent, which include a synthetic polymer and at least 10 mass%, on a total particle mass basis, of an aggregation-induced emission material, and support a binding partner for an analyte. As used herein, "supporting" a binding partner for an analyte on a particle may also be referred to as "binding".

The synthetic polymer constituting the fluorescent particles for a diagnostic agent is not particularly limited, but examples thereof may include polystyrene, a styrene-styrene sulfonate copolymer, a methacrylic acid polymer, an acrylic acid polymer, an itaconic acid polymer, a styrene-hydrophilic carboxyl monomer copolymer such as a styrene-methacrylic acid copolymer, a styrene-acrylic acid copolymer, and a styrene-itaconic acid copolymer. Among them, a styrene-methacrylic acid copolymer, a styrene-itaconic acid copolymer, a styrene and styrene-styrene sulfonate copolymer are preferred. A styrene and styrene-(meth)acrylic acid copolymer is particularly preferred.

The salt of styrene sulfonate is not particularly limited, and may include a sodium salt, a potassium salt, a lithium salt, an ammonium salt and the like. These may be used alone or in combination of two or more thereof. Among them, sodium styrene sulfonate is preferably used. As the hydrophilic carboxyl monomer used in the present invention, methacrylic acid, acrylic acid, itaconic acid, maleic acid, fumaric acid or the like may be used. Preferably, methacrylic acid and acrylic acid may be used.

When the hydrophilic carboxyl monomer is used, the amount of carboxyl groups possessed by the synthetic polymer after polymerization influences the light emission intensity of the fluorescent particles. Thus, when the amount of carboxyl groups on the particle surface is 0.001 meq/g to 0.6 meq/g, the fluorescent particles exhibit strong light emission depending on the amount of the carboxyl group. The amount of carboxyl groups on the particle surface is preferably 0.1 meq/g to 0.6 meq/g, and more preferably 0.2 meq/g to 0.6 meq/g. The amount of carboxyl groups on the particle surface may be measured by a common measurement method. For example, it may be measured using an automatic potentiometric titrator.

The aggregation-induced emission material constituting the fluorescent particles for a diagnostic agent is not particularly limited, but examples thereof may include ketoimine boron complex derivatives, diimine boron complex derivatives, tetraphenylethylene derivatives, aminomaleimide derivatives, aminobenzopyroxanthene derivatives, triphenylamine derivatives, hexaphenylbenzene derivatives, hexaphenylsilole derivatives and the like. Among the above-described derivatives, the tetraphenylethylene derivatives are preferred, because they are easy to synthesize and are also commercially available.

Examples of the tetraphenylethylene derivatives may include ethylene derivatives substituted with four or more phenyl groups or phenyl group derivatives. Specifically, an ethylene derivative represented by the following formula (1) may be mentioned: 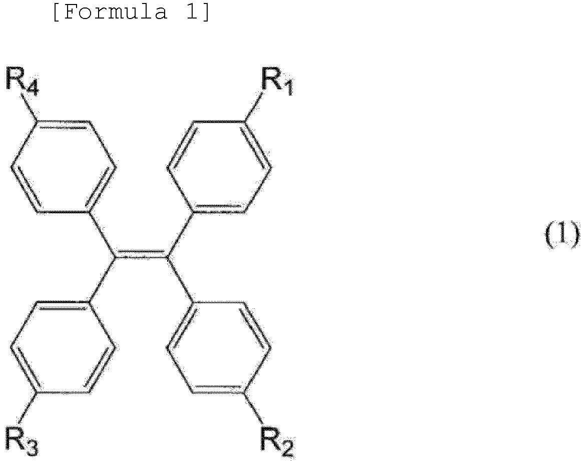 (wherein R₁ represents any one of a hydrogen atom, a bromine atom, and a hydroxyl group, and R₂, R₃ and R₄ represent a hydrogen atom or a hydroxyl group, respectively.)

More specifically, tetraphenylethylene, 1-(4-bromophenyl)-1,2,2-triphenylethylene, and tetrakis(4-hydroxyphenyl)ethylene may be mentioned.

Examples of the hexaphenylbenzene derivative may include benzene derivatives substituted with four or more phenyl groups or phenyl group derivatives. Specifically, hexaphenylsilole or hexaphenylbenzene may be mentioned.

Examples of the triphenylamine derivative may include 4-(di-p-triamino)benzaldehyde.

A number average molecular weight of the aggregation-induced emission material is preferably 10,000 or less. If the number average molecular weight exceeds the upper limit, the aggregation-induced emission material is hard to dissolve, and thus it may not be processed into a particle shape, or its content tends to decrease.

Besides being contained in itself in the fluorescent particles for a diagnostic agent, each of the above-mentioned derivatives may be included in the fluorescent particles for a diagnostic agent in the form of being incorporated into the main chain or side chain of the polymer. However, when the derivative is introduced into the polymer chain, the content decreases or a process of another step is required for particle synthesis, and therefore, it is preferable that each derivative in itself be contained in the fluorescent particles for a diagnostic agent.

A percentage of the aggregation-induced emission material on a total particle mass basis is 10 mass% or more, preferably 30 mass% or more, and preferably 95 mass% or less, from the viewpoint of improving visibility. When it exceeds 95 mass%, the CV value of the particle becomes large and reproducibility of an assay reagent may deteriorate.

The average particle diameter of the fluorescent particles for a diagnostic agent is not particularly limited. As long as colored latex particles for a diagnostic agent are used as the fluorescent particles for a diagnostic agent, they may have any average particle diameter. In particular, there is no particular problem as long as it is an average particle diameter that may be developed by chromatography, but if particles having a small particle diameter are used, sufficient detection sensitivity may not be obtained, and therefore, particles of 300 nm or more and 1000 nm or less are more preferred.

Further, as used herein, "average particle diameter" is calculated by observing the particles at a magnification enabling observation of about 100 aggregation-induced emission material-containing particles in one visual field with a scanning electron microscope (SEM), measuring the longest diameters of arbitrarily selected 50 aggregation-induced emission material-containing particles with a micrometer caliper, and calculating the number average value of the longest diameters.

A coefficient of variation (CV value) of the particle diameter of the particles is preferably 20% or less. If the CV value exceeds 20%, lot reproducibility during preparation of a reagent is poor, and reproducibility of the assay reagent may deteriorate. More preferably, the CV value is 15% or less. Further, the coefficient of variation of the particle diameter may be calculated according to the following formula.

Coefficient of variation (CV value) of particle diameter = standard deviation of particle diameter / average particle diameter

A method of preparing the fluorescent particles for a diagnostic agent is not particularly limited, and a known method may be used. However, a seed swelling method is preferably used, since particles having a uniform particle diameter may be obtained. The seed swelling method is a method of swelling styrene polymer particles having a uniform particle diameter, which are synthesized in advance, with a solution in which the aggregation-induced emission material is dissolved. Therefore, uniform droplets containing the aggregation-induced emission material are prepared and dried for an appropriate time, thereby obtaining intended fluorescent particles.

For example, when styrene and sodium styrene sulfonate and methacrylic acid as the hydrophilic carboxyl monomer are used, the obtained particles have a uniform particle size distribution and excellent dispersion stability. The dispersion stability is attributed to the electrostatic repulsive force between sulfonate groups derived from styrene sulfonate present on the surface of the respective fluorescent particles for a diagnostic agent. Further, it is preferable that carboxyl groups are present on the particle surface, because the carboxyl groups may contribute to dispersion stability and may also be used as a chemical binding site with an antibody.

### [Method of preparing fluorescent particles for diagnostic agent]

A method of preparing the fluorescent particles for a diagnostic agent is not particularly limited. An example may include the following method (a seed swelling method).
(i) First, a synthetic polymer constituting seed particles is prepared. A method of preparing the synthetic polymer is not particularly limited, and a known method may be used, but a soap-free emulsion polymerization method without using an emulsifier (surfactant) is preferred. A polymerization initiator used in the emulsion polymerization method may include potassium persulfate, ammonium persulfate and the like, preferably, potassium persulfate. In the present invention, ion exchange water, for example, a monomer and a polymerization initiator are charged in a reaction vessel, and the reaction vessel is purged with nitrogen under stirring, and then reaction is allowed at 65°C to 80°C for 12 hours to 42 hours. The obtained particles have a low CV value and excellent dispersion stability. As the synthetic polymer, the above-described synthetic polymer may be used. In this regard, from the viewpoint of increasing the degree of dispersion of the fluorescent particles for a diagnostic agent and enhancing the limitation of visibility evaluation, it is preferable that seed particles composed of the synthetic polymer have an average particle diameter of 0.05 µm to 3. 0 µm and the CV value of 1% to 20%.
(ii) Next, the obtained seed particles are dispersed in a solvent to prepare a seed particle dispersion. The solvent is not particularly limited, but water may be used.
(iii) An emulsion containing a solution prepared by dissolving the aggregation-induced emission material in an organic solvent is prepared. For example, the emulsion may be prepared by dissolving tetraphenylethylene, which is the aggregation-induced emission material, in ethyl acetate and adding the obtained tetraphenylethylene solution to an aqueous solution which is prepared by dissolving sodium styrene sulfonate in water.
(iv) The emulsion is added to the seed particle dispersion under stirring to obtain a swollen particle droplet dispersion. Thereafter, the solution obtained by adding the emulsion to the seed particle dispersion is continuously stirred for about 1 minute to about 36 hours, preferably for about 1 hour to about 30 hours, more preferably for about 12 hours to about 24 hours, and still more preferably for about 20 hours to about 24 hours to absorb the aggregation-induced emission material and the organic solvent into the seed particles. When the dispersion of the swollen particle droplets is obtained, the emulsion is preferably added such that the mass of the aggregation-induced emission material is 0.1 time or more and 64 times or less of the mass of the seed particles.
(v) The aggregation-induced emission material-containing particles are obtained by in-liquid drying the organic solvent in the swollen particle droplets. Conditions of the in-liquid drying are determined by a boiling point and a vaporization point of the organic solvent. For example, when ethyl acetate is used as the organic solvent, ethyl acetate may be dried by stirring the resultant dispersion of the swollen particle droplets at 65°C at a speed of 200 rpm for about 24 hours. Depressurization may also be performed in place of heating (or in combination with heating).

As described above, aggregation-induced emission material-containing particles are prepared.

### [Use of Particles]

The fluorescent particles for a diagnostic agent of the present invention are bound with, on the surface thereof, an antigen (or antibody) as a binding partner for an analyte, thereby being appropriately employed in various methods using biological reactions, such as an enzyme immunoassay method, a fluorescence immunoassay method, a latex agglutination method, or an immunochromatography method, these methods using an antigen-antibody reaction.

The present invention provides an immunoassay reagent using the above-described fluorescent particles for a diagnostic reagent.

A method of binding an antigen (or antibody) to the particle surface of the fluorescent particles for a diagnostic agent is not particularly limited, and a conventional known method may be used. For example, a binding method by physical adsorption such as immersion of fluorescent particles for a diagnostic agent in a buffer solution containing an antigen (or antibody) and incubation for a predetermined time at a predetermined temperature, or a binding method by chemical adsorption may be used. The physical adsorption utilizes a hydrophobic interaction between the particle surface and the antigen (or antibody), and has an advantage of easy operation, and the chemical binding utilizes a chemical reaction between a reactive functional group on the particle surface and a specific group in the antigen (or antibody), and has an advantage that it is possible to control a distance between the particle and the antigen (or antibody) . When the synthetic polymer has a carboxyl group, the amino group contained in the antigen (or antibody) may be crosslinked and allowed to bind. These may be appropriately selected in consideration of characteristics of the binding partner for the analyte.

According to the present invention, it is possible to prepare fluorescent particles for a diagnostic agent which exhibit sufficiently strong light emission, and when the fluorescent particles for a diagnostic agent are used as an immunoassay reagent, visual judgment may be remarkably improved and detection sensitivity may be improved. Further, since the degree of particle dispersion is low, lot reproducibility during preparation of the reagent is improved.

Specific uses of the fluorescent particles for a diagnostic agent and terminology will be described below.

### [Analyte Measuring Method]

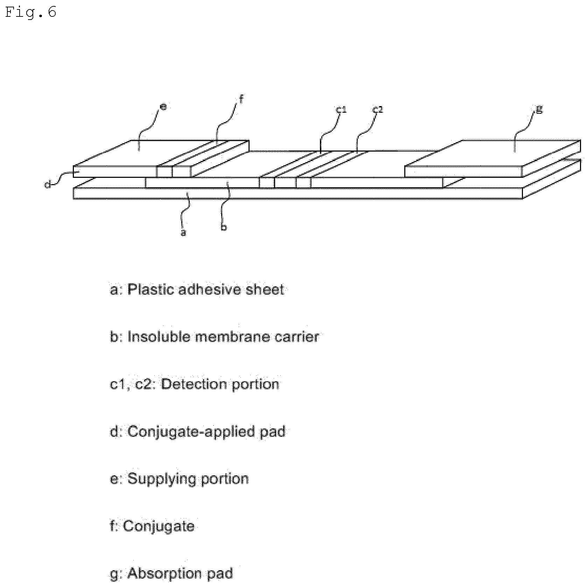

According to the present invention, provided is an analyte measuring method including a step of preparing a mixed solution by mixing a sample solution containing or possibly containing an analyte with a solution containing aggregation-induced emission fluorescent material-containing particles that have a binding partner for the analyte (including the case where the solution exists in a dry state in a so-called conjugate-applied pad as shown in FIG. 6f); a step of developing the mixed solution on an insoluble carrier having at least one detection portion to which a binding partner for an analyte is immobilized; a step of measuring fluorescence intensity generated from the aggregation-induced emission fluorescent material-containing particles in the detection portion; and a step of comparing a fluorescence intensity calibration curve for an analyte concentration with the fluorescence intensity, and associating the fluorescence intensity with the analyte concentration in the mixed solution (The step of associating the fluorescence intensity with the analyte concentration in the mixed solution includes a qualitative or quantitative association for determining the presence or absence of the analyte according to the intensity relationship with a reference fluorescence intensity).

According to this measuring method, the presence or absence of the analyte and the analyte concentration may be accurately measured by using the aggregation-induced emission fluorescent material-containing particles with high sensitivity, even when the analyte concentration is low. The association may be performed visually or by means of an apparatus. In the case of visual observation, the present invention is particularly advantageous.

Further, the analyte measurement may be performed over a wide range using an existing measuring device by using a measuring reagent consisting of a first reagent solution (R1) and a second reagent solution (R2) described below in an appropriate combination.

### (Test strip)

According to the present invention, provided is an immunochromatographic test strip, as shown in FIG. 6, including a plastic adhesive sheet a; an insoluble membrane carrier b disposed on the plastic adhesive sheet, the insoluble membrane carrier b having at least one detection portion c on which binding partners for an analyte are immobilized; a supplying portion e of a sample solution possibly containing an analyte, which is disposed at one end of the insoluble membrane carrier b; a conjugate-applied pad d having a conjugate f on which aggregation-induced emission fluorescent material-containing particles having binding partners for the analyte are immobilized; and an absorption pad g which is disposed at the other end of the insoluble membrane carrier b.

According to this test strip, use of the above-described highly sensitive aggregation-induced emission fluorescent material-containing particles enables very easy visual confirmation, as compared with conventional test strips.

Further, a plurality of the detection portions may be provided, as shown in FIG. 6, thereby testing other items may be possible. As in FIG. 6, in order to shorten the measurement time and to improve the visibility, the conjugate f is formed in a line shape in a region where the rear surface of the conjugate-applied pad d is brought into contact with the surface of the insoluble membrane carrier b, excluding the downstream side end in a flow direction of the sample. However, the arrangement position or shape of the conjugate f is not particularly limited.

### (Sample)

An object to be measured is not particularly limited, but various biological samples may be mentioned. For example, it is a body fluid such as blood, serum, plasma, urine, saliva, sputum, nasal discharge, nasal swab, pharyngeal swab, and tear.

### (Analyte)

The analyte is not particularly limited as long as it is a molecule which may be theoretically measured with a limit of the existence of a partner binding to the analyte, such as proteins, peptides, amino acids, lipids, sugars, nucleic acids, and haptens. Examples thereof may include viruses such as influenza virus, bacteria, CRP (C reactive protein), Lp (a) (lipoprotein (a)), MMP 3 (matrix metalloproteinase 3), anti-CCP (cyclic citrullinated peptide) antibody, anti-phospholipid antibody, anti-syphilis antigen antibody, RPR, type IV collagen, PSA, AFP, CEA, BNP (brain natriuretic peptide), NT-proBNP, insulin, microalbumin, cystatin C, RF (rheumatoid factor), CA-RF, KL-6, PIVKA-II, FDP, D-dimer, SF (soluble fibrin), TAT (thrombin-antithrombin III complex), PIC, PAI, factor XIII, pepsinogen I, pepsinogen II, phenytoin, phenobarbital, carbamazepine, valproic acid, theophylline and the like.

### (Binding Partner)

The binding partner may include proteins, peptides, amino acids, lipids, sugars, nucleic acids, haptens, or the like which is a material binding to an analyte, but antibodies and antigens are generally used, in view of their specificity and affinity. Further, as long as the binding specificity and affinity for the analyte are within the desired range, there are no particular limitation in its molecular weight (e.g., in the case of antibodies, either whole immunoglobulins or analyte-binding functional fragments) and origin, either naturally occurring or being synthesized (e.g., in the case of antibodies, either those derived from animal body fluids or those by genetic recombination technology).

### (Assay Reagent)

A composition of the assay reagent which is provided in the measuring method of the present invention is not particularly limited, but a reagent for immunochromatography, including the above-described test strip, is preferred. Considering use of the reagent in an automated analyzer generally used in the field of clinical tests, the assay reagent is generally composed of two solutions of a first reagent solution (R1) containing a buffer solution and a second reagent solution (R2) containing aggregation-induced emission material-containing particles which support binding partners for the analyte.

### (Components of Assay Reagent)

In addition to the microparticles supporting binding partners which are a main component for reaction, components of the assay reagent using the aggregation-induced emission fluorescent material-containing particles of the present invention may include a component for buffering the ionic strength or osmotic pressure of a sample, for example, acetic acid, citric acid, phosphoric acid, Tris, glycine, boric acid, carbonic acid, Good's buffer, and sodium salts, potassium salts, and calcium salts thereof, etc. In addition, polymers such as polyethylene glycol, polyvinylpyrrolidone, and phospholipid polymers may be contained as a component for promoting and enhancing the binding (for example, antigen-antibody reaction) between the analyte and the partner binding to the analyte . One or more of generally used components, such as polymer materials, proteins, amino acids, sugars, metal salts, surfactants, reducing substances, and chaotropic substances may also be contained in combination as a component for controlling the binding between the analyte and the partner binding to the analyte. An antifoaming substance may also be contained.

### EXAMPLES

Hereinafter, the present invention will be described in more detail with reference to Examples and Comparative Examples . However, the present invention is not limited thereto.

### (Example 1)

### (Preparation of Seed Particles)

400 g of ultrapure water, 10 g of styrene monomer, and 0.20 g of potassium persulfate were added to a glass reaction vessel (1 L volume) equipped with a stirrer, a reflux condenser, a thermometer, a nitrogen inlet tube, and a jacket, and the vessel was purged with nitrogen, and polymerization was allowed for 24 hours at 65°C under stirring at a speed of 200 rpm (a soap-free emulsion polymerization method).

After completion of the polymerization, the above solution was filtrated with a paper filter, and latex particles were extracted. Thereafter, dialysis treatment was performed with a dialysis membrane for 48 hours to obtain purified latex particles for an assay reagent as seed particles. An average particle diameter of the obtained latex particles was 0.15 µm, and a CV value of the particle diameter was 5.1%.

### (Preparation of Aggregation-induced Emission material-Containing Particles)

A solution prepared by dissolving 0.59 g of hexaphenylsilole as an aggregation-induced emission material in 31 g of ethyl acetate was added to and mixed with an aqueous solution prepared by dissolving 0.1 g of sodium styrene sulfonate in 150 g of water, thereby preparing an emulsion.

The emulsion was added to the seed particle dispersion synthesized as described above such that the mass of hexaphenylsilole was 0. 43 times the mass of the seed particles, and stirred for 24 hours to obtain a swollen particle droplet dispersion of seed particles that absorbed hexaphenylsilole and ethyl acetate.

The ethyl acetate was dried while stirring the obtained swollen particle droplet dispersion at 65°C and a speed of 200 rpm for 24 hours to obtain aggregation-induced emission material-containing particles (a seed swelling method).

### (Example 2)

Aggregation-induced emission material-containing particles were obtained in the same manner as in Example 1, except that the hexaphenylsilole was changed to hexaphenylbenzene.

### (Example 3)

Aggregation-induced emission material-containing particles were obtained in the same manner as in Example 1, except that the hexaphenylsilole was changed to 4-(di-p-tolylamino)benzaldehyde.

### (Example 4)

Aggregation-induced emission material-containing particles were obtained in the same manner as in Example 1, except that the hexaphenylsilole was changed to tetraphenylethylene of 0.11 times the mass of seed particles.

### (Example 5)

Aggregation-induced emission material-containing particles were obtained in the same manner as in Example 1, except that the hexaphenylsilole was changed to tetraphenylethylene.

### (Example 6)

Aggregation-induced emission material-containing particles were obtained in the same manner as in Example 1, except that the hexaphenylsilole was changed to tetraphenylethylene of the equivalent mass to the seed particles.

### (Example 7)

Aggregation-induced emission material-containing particles were obtained in the same manner as in Example 1, except that the hexaphenylsilole was changed to tetraphenylethylene of 2.33 times the mass of seed particles.

### (Example 8)

Aggregation-induced emission material-containing particles were obtained in the same manner as in Example 1, except that the hexaphenylsilole was changed to tetraphenylethylene of 9 times the mass of seed particles.

### (Example 9)

### (Preparation of Seed Particles)

400 g of ultrapure water, 10 g of styrene monomer, 1 g of methacrylic acid, and 0.20 g of potassium persulfate were added to a glass reaction vessel (1 L volume) equipped with a stirrer, a reflux condenser, a thermometer, a nitrogen inlet tube, and a jacket, and the vessel was purged with nitrogen, and polymerization was allowed for 24 hours at 65°C under stirring at a speed of 200 rpm (a soap-free emulsion polymerization method).

After completion of the polymerization, the above solution was filtrated with a paper filter, and latex particles were extracted. Thereafter, dialysis treatment was performed with a dialysis membrane for 48 hours to obtain purified latex particles for an assay reagent as seed particles. An average particle diameter of the obtained latex particles was 0.16 µm, and a CV value of the particle diameter was 8.6%.

### (Preparation of Aggregation-induced Emission material-Containing Particles)

A solution prepared by dissolving 0.59 g of tetraphenylethylene as an aggregation-induced emission material in 31 g of ethyl acetate was added to and mixed with an aqueous solution prepared by dissolving 0.1 g of sodium styrene sulfonate in 150 g of water, thereby preparing an emulsion.

The emulsion was added to the seed particle dispersion synthesized as described above such that the mass of tetraphenylethylene was 0.11 times the mass of the seed particles, and stirred for 24 hours to obtain a swollen particle droplet dispersion of seed particles that absorbed tetraphenylethylene and ethyl acetate.

The ethyl acetate was dried while stirring the obtained swollen particle droplet dispersion at 65°C and a speed of 200 rpm for 24 hours to obtain aggregation-induced emission material-containing particles (a seed swelling method).

### (Example 10)

Aggregation-induced emission material-containing particles were obtained in the same manner as in Example 1, except that the hexaphenylsilole was changed to 1-(4-bromo phenyl)-1,2,2-triphenyl ethylene.

### (Example 11)

Aggregation-induced emission material-containing particles were obtained in the same manner as in Example 1, except that the hexaphenylsilole was changed to tetrakis(4-hydroxyphenyl)ethylene.

### (Comparative Example 1)

Aggregation-induced emission material-containing particles were obtained in the same manner as in Example 4, except that the hexaphenylsilole was changed to 0.05 times the mass of seed particles.

### (Comparative Example 2)

### (Preparation of Seed Particles)

10 g of styrene monomer in which 0.01 g of azoisobutyronitrile was dissolved was added to 400 g of ultrapure water in which 0.1 g of sodium styrene sulfonate was dissolved, and this mixture was stirred using a homogenizer at 12000 rpm for 5 min to obtain an emulsion. The obtained emulsion was added to a glass reaction vessel (1 L volume) equipped with a stirrer, a reflux condenser, a thermometer, a nitrogen inlet tube, and a jacket, and polymerization was allowed for 24 hours at 65°C under stirring at a speed of 200 rpm (suspension polymerization).

After completion of the polymerization, the above solution was filtrated with a paper filter, and latex particles were extracted. Thereafter, dialysis treatment was performed with a dialysis membrane for 48 hours to obtain purified latex particles for an assay reagent as seed particles. An average particle diameter of the obtained latex particles was 0.22 µm, and a CV value of the particle diameter was 34.5%.

### (Preparation of Aggregation-induced Emission material-Containing Particles)

A solution prepared by dissolving 0.59 g of tetraphenylethylene as an aggregation-induced emission material in 31 g of ethyl acetate was added to and mixed with an aqueous solution prepared by dissolving 0.1 g of sodium styrene sulfonate in 150 g of water, thereby preparing an emulsion.

The emulsion was added to the seed particle dispersion synthesized as described above such that the mass of tetraphenylethylene was 0.11 times the mass of the seed particles, and stirred for 24 hours to obtain a swollen particle droplet dispersion of seed particles that absorbed tetraphenylethylene and ethyl acetate.

The ethyl acetate was dried while stirring the obtained swollen particle droplet dispersion at 65°C and a speed of 200 rpm for 24 hours to obtain aggregation-induced emission material-containing particles.

**[Table 1]**

| | Kind of aggregation-induced emission material | Content rate of aggregation-induced emission material (%) | Composition of synthetic polymer | Content of synthetic polymer | CV value (%) | Visibility evaluation | Reproducibility |
|---|---|---|---|---|---|---|---|
| Example 1 | Hexaphenylsilole | 30 | Styrene | 70 | 10 | 5 | ⊚ |
| Example 2 | Hexaphenylbenzene | 30 | Styrene | 70 | 10 | 5 | ⊚ |
| Example 3 | 4-(di-p-tolylamino)benzaldehyde | 30 | Styrene | 70 | 10 | 4 | ⊚ |
| Example 4 | Tetraphenylethylene | 10 | Styrene | 90 | 10 | 4 | ⊚ |
| Example 5 | Tetraphenylethylene | 30 | Styrene | 70 | 10 | 5 | ⊚ |
| Example 6 | Tetraphenylethylene | 50 | Styrene | 50 | 10 | 5 | ⊚ |
| Example 7 | Tetraphenylethylene | 70 | Styrene | 30 | 10 | 5 | ⊚ |
| Example 8 | Tetraphenylethylene | 90 | Styrene | 10 | 18 | 5 | ○ |
| Example 9 | Tetraphenylethylene | 10 | Styrene + Carboxylic acid | 90 | 10 | 4 | ⊚ |
| Example 10 | 1-(4-Bromophenyl)-1,2,2-triphenyl ethylene | 30 | Styrene | 30 | 10 | 5 | ⊚ |
| Example 11 | Tetrakis(4-hydroxyphenyl)ethylene | 30 | Styrene | 30 | 10 | 4 | ⊚ |
| Comparative Example 1 | Tetraphenylethylene | 5 | Styrene | 95 | 6.4 | 1 | ⊚ |
| Comparative Example 2 | Tetraphenylethylene | 10 | Styrene | 90 | 25 | 4 | × |

### (1) Measurement of Average Particle Diameter and Dispersion Degree

A scanning electron microscope (SEM) was used to observe particles at a magnification capable of observing approximately 100 aggregation-induced emission material-containing particles in one visual field, and the longest diameters of arbitrarily selected 50 aggregation-induced emission material-containing particles were measured with a micrometer caliper, and the number average value and coefficient of variation of the values were determined and taken as the average particle diameter and the dispersion degree.

### (2) Content Rate of Aggregation-induced emission material

The content (mass) of the aggregation-induced emission material contained in 0.01 g of the aggregation-induced emission material-containing particles was measured using pyrolysis gas chromatography (Q 1000, manufactured by JEOL Ltd.) .

### (3) Content of Synthetic Polymer

The content of the synthetic polymer contained in 0.01 g of the aggregation-induced emission material-containing particles was measured using pyrolysis gas chromatography (Q 1000, manufactured by JEOL Ltd.).

### (4) Visibility

13 kinds of the immunochromatographic reagents for measuring influenza virus which were prepared according to "1"∼ "5" in the following Application Example were used, and influenza A virus which was adjusted to 3.8 × 10⁵ to 4.8 × 10⁵ TCID_{50/}mL by a sample extraction solution described in column "6" was used as a sample to evaluate visibility according to "8. Evaluation and Measurement of Reagent performance".

### (5) Reproducibility

The visibility evaluation was repeated three times. As a result, when all were consistent, it was evaluated as ⊚, when there was 1 or less error, it was evaluated as ○, and when there were 2 or more errors, it was evaluated as ×.

### (6) Surface observation

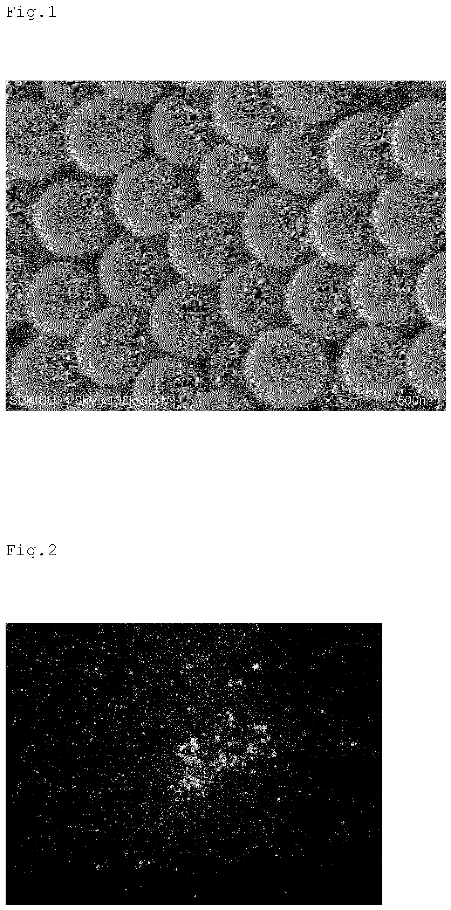

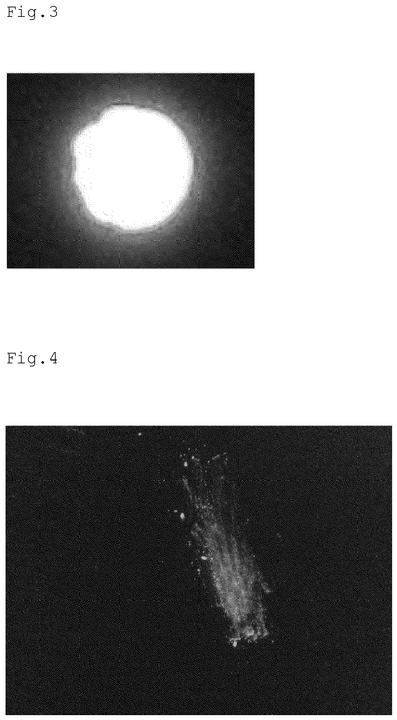

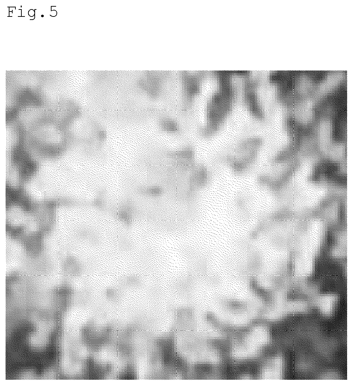

The surface of the aggregation-induced emission material-containing particles was observed using a scanning electron microscope (SEM). Among obtained SEM photographs, an SEM photograph of Example 10 is shown in FIG. 1. In addition, FIG. 2 shows aggregating luminescence when the aggregation-induced emission material-containing particles of the present invention were irradiated with ultraviolet rays (UV) of a wavelength of 365 nm. FIG. 3 is a partially enlarged view of FIG. 2. FIG. 4 shows aggregating luminescence when aggregation-induced emission material-containing particles of Comparative Example 1 were irradiated with ultraviolet rays (UV) of a wavelength of 365 nm. FIG. 5 is a partially enlarged view of FIG. 4.

### [Application Example]

### <Preparation of Immunochromatographic Reagent for Measuring Influenza Virus>

### 1. Preparation of Aggregation-induced emission material-Containing particle-Labeled Anti-Influenza A Virus Antibody

(1) The following (i) to (iv) were prepared, and 5 mL of (i), 0.2 mL of (ii), and 0.8 mL of (iii) were added and stirred, and then 4 mL of (iv) was added thereto, and stirred at room temperature for 2 hours.
(2) The solution obtained in the above (1) was centrifuged at 13,000 rpm for 10 minutes, the resulting supernatant was removed, and 10 mL of a 2% bovine serum albumin (BSA) aqueous solution containing 10% sucrose was added, and stirred for 2 hours, and then centrifuged at 13,000 rpm for 10 minutes to obtain 13 kinds of conjugates.
(3) 10 mL of a 2% BSA aqueous solution containing 10% sucrose was added to the conjugate obtained in the above (2), and the conjugate was suspended to obtain a blue colored latex particle-labeled anti-influenza A virus antibody.
   (i) 20 mM MES buffer solution (pH 6.5) containing each of 2% blue aggregation-induced emission material-containing particles (13 kinds in total from Examples 1 to 11 and Comparative Examples 1 and 2)
   (ii) 20 mM MES buffer solution (pH 6.5)
   (iii) 15 mg/mL 1-ethyl-3-[3-(dimethylamino) propyl] carbodiimide (EDC) as a crosslinking agent
   (iv) In reagent performance evaluation of 20 mM MES buffer solution (pH 6.5) containing 2.5 mg/mL anti-influenza A virus monoclonal antibody, absorbance of the conjugate was measured at the maximum absorption wavelength of the aggregation-induced emission material.

### 3. Fabrication of Conjugate-Applied Pad

Each of 13 kinds of the conjugates prepared in the above 1 and 2 was mixed with a Tris buffer solution (pH 8.5) containing 0.5% casein and 10% sucrose so as to be 6.4 OD/mL, thereby preparing respective conjugate solutions. Next, a glass fiber pad (manufactured by Lydall) having 22.0 mm × 254 mm × 0.56 mm (width × length × thickness) was soaked with 10 µL/cm of the conjugate solution using a dispenser "XUZ 3050" (manufactured by BIO DOT) for immunochromatography. Thereafter, the pad was heated and dried in a dry oven at 70°C for 30 minutes to obtain a conjugate-applied pad. When a surfactant (e.g., Emulgen 150 (manufactured by Kao Corp.), Amito 320 (manufactured by Kao Corp.)) is added, a necessary amount thereof is added to the above conjugate solution, and the same operation may be carried out.

### 4. Fabrication of Anti-Influenza Virus Antibody-Immobilized Membrane

To a nitrocellulose membrane (manufactured by Sartorius) of 25. 0 mm × 254 mm × 0.235 mm (short side × long side × thickness), an anti-influenza A virus antibody which has an epitope different from that of the above blue colored latex particle-labeled anti-influenza A virus antibody and was prepared at 0.75 mg/mL, an anti-KLH antibody prepared at 0.75 mg/mL, and a 10 mM phosphate buffer solution (pH 7.2) containing 2.5% sucrose were applied in a line shape having a width of about 1 mm. The application was performed using a dispenser "XYZ3050" (manufactured by BIO DOT) for immunochromatography, and the discharge amount was set to be 1 µL/cm. After applying the line, the nitrocellulose membrane was dried in a dry oven at 70°C for 45 minutes to fabricate an anti-influenza virus antibody-immobilized membrane.

### 5. Fabrication of Test Strip

The anti-influenza virus antibody-immobilized membrane was attached to a plastic adhesive sheet, and 13 kinds of the conjugate-applied pads prepared in the above 3 were respectively arranged and mounted, and on the opposite end, an absorption pad (manufactured by Whatman, 740 E) was arranged and mounted (see FIG. 6). Finally, a polyester film was arranged and mounted, laminated on the upper surface so as to cover the antibody-immobilized membrane and the absorption pad. A structure obtained by superposing the respective components in this manner was cut into a width of 4 mm to fabricate each test strip. The size of the test strip was 4 mm × 98 mm (width × length) and was in the form of an immunochromatographic test strip.

### 6. Preparation of Sample Extraction Solution

A 50 mM Tris buffer solution (pH 8.5) containing 200 mM potassium chloride, 150 mM arginine, 0.5% Brij 35, 0.25% BSA, and 0.05% ProClin (registered trademark) 950 was used as a sample extraction solution.

### 7. Sample Preparation

### a. In case of nasal aspiration sample

One cotton swab was immersed in a nasal aspirate, and the cotton swab impregnated with the sample was put in 320 µL of PBS to allow the sample components to be dissolved in PBS, thereby preparing a sample for evaluation of reagent performance. In this regard, the concentration of influenza A virus was equivalent to 3.8 × 10⁵ to 4.8 × 10⁵ TCID_{5C}/mL.

### b. In case of nasal swab sample

Nasal cavity was wiped with two cotton swabs, and the swabs were dissolved in 320 µL of PBS to prepare samples for evaluation of reagent performance. In this regard, the concentration of influenza A virus was equivalent to 3.8 × 10⁵ to 4.8 × 10⁵ TCID₅₀/mL.

### 8. Evaluation and Measurement of Reagent performance

13 kinds of the test strips fabricated in the above 5 were immersed in the sample, and 10 minutes later, UV light was irradiated to A line (detecting portion C1) and the control line (not shown), and the light emission intensity was measured. The light emission intensity (visibility) was evaluated in 5 stages, and measurement was performed with n = 3. The same results as in the visibility of Table 1 were obtained.

### INDUSTRIAL APPLICABILITY

Fluorescent particles for a diagnostic agent of the present invention and an immunoassay reagent using the same contribute to early diagnosis of diseases and prevention of misjudgment because of excellent visual judgment and detection sensitivity when used as particles for immunoassay such as immunochromatography. Also, if the measurement sensitivity is given at about the conventional level, the amount of antibodies to be used may be reduced, usefully leading to cost reduction.

## Claims

1. Fluorescent particles for a diagnostic agent comprising:
a synthetic polymer; and
at least 10 mass%, on a total particle mass basis, of an aggregation-induced emission material;
wherein the fluorescent particles have on the surface thereof a binding partner binding with an analyte.

2. The fluorescent particles for a diagnostic agent of claim 1, wherein the particles have a CV value of 20% or less and an average particle diameter of 0.05 µm to 3.0 µm.

3. The fluorescent particles for a diagnostic agent of claim 1 or 2, wherein the synthetic polymer includes at least one selected from the group consisting of styrene and styrene-(meth)acrylic acid copolymer.

4. The fluorescent particles for a diagnostic agent of any one of claims 1 to 3, wherein a number average molecular weight of the aggregation-induced emission material is 10, 000 or less.

5. The fluorescent particles for a diagnostic agent of any one of claims 1 to 4, wherein the aggregation-induced emission material is an ethylene derivative or a benzene derivative substituted with four or more phenyl groups or phenyl group derivatives.

6. The fluorescent particles for a diagnostic agent of any one of claims 1 to 4, wherein the aggregation-induced emission material is at least one selected from the group consisting of tetraphenylethylene, 1-(4-bromophenyl)-1,2,2-triphenyl ethylene, and tetrakis(4-hydroxyphenyl)ethylene.

7. The fluorescent particles for a diagnostic agent of any one of claims 1 to 4, wherein the aggregation-induced emission material is hexaphenylsilole and/or hexaphenylbenzene.

8. An immunoassay reagent using the fluorescent particles for a diagnostic agent of any one of claims 1 to 7.

9. A method of preparing aggregation-induced emission material-containing particles comprising:
preparing a seed particle dispersion in which seed particles containing a synthetic polymer are dispersed,
preparing an emulsion containing an aggregation-induced emission material and an organic solvent,
obtaining a swollen particle droplet dispersion by adding the emulsion to the seed particle dispersion and absorbing the aggregation-induced emission material and the organic solvent into the seed particles, and
obtaining the aggregation-induced emission material-containing particles by in-liquid drying the organic solvent in the swollen particle droplets.

10. The method of preparing aggregation-induced emission material-containing particles of claim 9, wherein the emulsion is added to the seed particle dispersion such that the amounts of the aggregation-induced emission material and the organic solvent are 0.1 time to 64 times the mass of the seed particles.

11. A method of measuring an analyte comprising:
preparing a mixed solution by mixing a sample solution possibly containing an analyte and a solution containing aggregation-induced emission fluorescent material-containing particles having a binding partner for the analyte;
developing the mixed solution on an insoluble carrier having at least one detection portion on which binding partners for the analyte are immobilized;
measuring the fluorescence intensity generated from the aggregation-induced emission fluorescent material-containing particles in the detection portion; and
comparing a fluorescence intensity calibration curve for an analyte concentration with the fluorescence intensity, and associating the fluorescence intensity with the analyte concentration in the mixed solution.

12. An immunochromatographic test strip comprising:
(a) a supplying portion of a sample solution that possibly contains an analyte,
(b) a conjugate pad that contains aggregation-induced emission fluorescent material-containing particles having on the surface thereof binding partners for the analyte, and
(c) an insoluble membrane carrier having at least one detection portion on which binding partners for the analyte are immobilized, wherein the aggregation-induced emission fluorescent material-containing particles contain (i) a synthetic polymer and at least 10 mass%, on a total particle mass basis, of (ii) an aggregation-induced emission material.

13. The test strip of claim 12, wherein the aggregation-induced emission fluorescent material-containing particles have a CV value of 20% or less and an average particle diameter of 0.05 µm to 3.0 µm.

14. The test strip of claim 12 or 13, wherein the synthetic polymer contained in the the aggregation-induced emission fluorescent material-containing particles is a copolymer including at least one selected from the group consisting of a styrene monomer and (meth)acrylic acid.

15. The test strip of any one of claims 12 to 14, wherein a number average molecular weight of the aggregation-induced emission material contained in the aggregation-induced emission fluorescent material-containing particles is 10,000 or less.

16. The test strip of any one of claims 12 to 15, wherein the aggregation-induced emission material contained in the aggregation-induced emission fluorescent material-containing particles is an ethylene derivative or a benzene derivative substituted with four or more phenyl groups or phenyl group derivatives.

17. The test strip of any one of claims 12 to 16, wherein the aggregation-induced emission material contained in the aggregation-induced emission fluorescent material-containing particles is selected from the group consisting of tetraphenylethylene, 1-(4-bromophenyl)-1,2,2-triphenyl ethylene, and tetrakis(4-hydroxyphenyl)ethylene.

18. The test strip of any one of claims 12 to 17, wherein the aggregation-induced emission material contained in the aggregation-induced emission fluorescent material-containing particles is hexaphenylsilole or hexaphenylbenzene.
